# EUROPEAN PATENT APPLICATION

(11) **EP 1 151 733 A2**
(43) Date of publication of application: **07.11.2001**
(21) Application number: 01110477.5
(22) Date of filing: 27.04.2001
(51) Int. Cl.: A61F 9/02

(54) **Ventilated, no-fogging goggles for sporting activities, particulary for winter sports, motorcyclists and the like**

(30) Priority: 03.05.2000 IT MI000956
(71) Applicant: O-NAMI PRODUCT DESIGN DI LUCA GAFFORIO & C. S.a.s., 24040 COMUN NUOVO (BG) (IT)
(72) Inventor: Gafforio, Luca, O-NAMI PRODUCT DESIGN DI LUCA, 24040 Comun Nuovo (BG) (IT); Cariani, Alessandro, O-NAMI PRODUCT DESIGN DI LUCA, 24040 Comun Nuovo (BG) (IT)
(74) Representative: Trupiano, Federica

(57) **Abstract**

Anti-misting (no-fogging) ventilated mask-type goggle for sporting activities, particularly for winter sports, such as skiing, cross-country skiing, snowboarding and the like, and for protective use in general, comprising at least one inlet opening (7) set at the front for the ventilation air and at least one outlet opening (6) for the ventilation air also set at the front and substantially on the same plane as said inlet opening, said outlet opening (6) being provided with a spoiler-shaped flap (4) which, in combination with the conformation of the external surface of the goggles in the proximity of said outlet opening (6) is designed to convey the flow of incident air so as to create a negative pressure at said outlet opening (6) and a consequent suction from the space inside the goggles outwards.

## Description

Forming the subject of the present invention are ventilated and anti-misting (no-fogging) mask-type goggles for sporting activities, particularly for winter sports, such as downhill skiing, cross-country skiing, snowboarding and the like, as well as for motorcycling and similar sports, and for protective use in general.

As is known, for sporting activities, such as motorcycling, skiing and the like, mask-type goggles are used for protecting the upper part of the user's face, including the eyes, from the wind and atmospheric agents, such as rain, snow, dust and so forth.

Mask-type protective goggles of this sort are made up basically of a frame made of elastically compliant material, such as rubber, plastic or similar materials, which surrounds the area of the user's eyes between the forehead, temples, cheek-bones and the root of the nose, the said frame being made to adhere to the face by means of a special strap, and of a transparent visor mounted, at times removably and in an interchangeable way, on said frame. There is usually associated to the frame a sealing element made of soft, comfortable material.

If is also known that mask-type goggles of this sort present the drawback of getting steamed up on account of the water vapour which is generated by the perspiration of the skin and which condenses in the form of drops on the internal surface of the transparent visor, so reducing and frequently practically blotting out visibility for the user, in particular when he or she is making an intense effort and/or when the temperature outside the goggles is considerably lower than the temperature existing in the space inside the goggles between the visor and the face of the user.

To overcome this problem various solutions are currently adopted.

For example, openings are made in the upper edge of the frame so that the hot air saturated with humidity, generated by perspiration, may come out upwards before condensing and steaming up the visor. This solution, however, proves of scant effectiveness at very low temperatures and when, for various reasons, the formation of the condensate is very high.

According to another solution openings are made at the front in the frame, through which, owing to the pressure that is created on the goggles as a result of the movement of the user, air from outside is introduced into the space inside the goggles, the said air then flowing out through other openings made at the two sides of the goggles, for example in the left and right temporal areas, thus producing a ventilation and consequent de-misting of the visor. In this case the intensity of the ventilation and hence the effectiveness of the de-misting action depend upon the movement of the user, in particular upon his speed, as well as upon the orientation of his head, and consequently of the goggles, with respect to his direction of advance. According to this solution, the higher the speed of the user, the greater the pressure of the air on the goggles and hence the greater the amount of air entering the internal space and bringing about the effect of de-misting the goggles. However, it follows that, at high speeds, the flow of air that penetrates into the internal space creates turbulence which causes watering of the eyes and some degree of discomfort, whilst at low speeds, for example at speeds lower than 10-15 km/h, and in particular when the user is about to stop or is already moving but has not yet reached an adequate speed, the air that penetrates into the space inside the goggles is insufficient, and consequently there is no de-misting at all, or else de-misting is very slow. Also in the case where the user is moving forward with his body, in particular with his head turned at a certain angle with respect to the direction of movement, as normally occurs in the case of downhill snowboarding, where the body and the head of the user are turned with respect to the direction of movement, the solution of making slits or notches in the front of the frame of the goggles is ineffective. In this case, in fact, the external air tends merely to lap the surface of the goggles, without generating variations of pressure and hence air flow in the aeration ducts or passages. In addition, the openings for outlet of the flow of air set on the sides of the frame in the temporal areas are subjected alternately to dynamic pressure, this pressure creating an obstacle which hinders the air from coming out, so that the air is directed prevalently towards the opening made on the opposite side, thus creating turbulence and lack of uniformity of ventilation which is troublesome and unpleasant for the user.

To overcome the above problems, goggles have been made in which, in addition to the ventilation slits or notches, there are provided two visors set on top of one another between which a gap is left that isolates the internal space from the external space. In this way, there is a reduction and practical elimination of the problem of misting. It is, however, obvious that this solution involves very high costs of manufacture, and moreover increases the overall dimensions and hence the degree of discomfort for the user.

One purpose of the present invention is to provide mask-type goggles for sporting activities, such as motorcycling, winter sports and the like, but also for protective purposes in general, which are ventilated and anti-misting (no-fogging), but free from the drawbacks mentioned above.

Another purpose of the invention is to provide mask-type goggles which are structured so that misting of the visor does not occur even when the user is travelling at very low speeds, practically even lower than 5 km/h, and which can thus be advantageously used also for sporting activities that involve slow movements, such as cross-country skiing, and which are moreover provided with an effective de-misting system in the case of marked deceleration.

A further purpose is that of providing mask-type goggles which are structured so that ventilation and de-misting are constant, effective and uniformly distributed even when the angle of the goggles varies with respect to the direction of movement of the user, and which are hence advantageously usable, in particular, for snowboarding.

These and yet other purposes and corresponding advantages that will emerge from the following description, are achieved by ventilated anti-misting (no-fogging) mask-type goggles for sporting activities in general, particularly for winter sports, such as skiing, cross-country skiing, snowboarding and the like and for protective use in general, comprising a frame and a visor applied to said frame also in such a way that it can be removed, the said goggles according to the present invention comprising at least one inlet opening set at the front for the incoming ventilation air and at least one outlet opening for the outgoing ventilation air, which is also set at the front and is basically on the same plane that comprises said inlet opening, said outlet opening being provided with a spoiler-shaped flap which, in combination with the conformation of the external surface of the goggles in the vicinity of said outlet opening, is designed to convey the flow of incident air so as to create a negative pressure at said outlet opening and a suction effect from the space inside the goggles outwards.

According to a preferred embodiment, said inlet opening is positioned in the top edge of said frame whilst said outlet opening or plurality of outlet openings provided with spoiler is positioned in the bottom edge of said frame.

According to another embodiment, said inlet opening is positioned in the bottom edge of said frame, whilst said outlet opening provided with spoiler is positioned in the top edge of said frame.

In addition, said outlet opening is preferably made up of a plurality of holes or slits or notches arranged symmetrically with respect to the middle of the goggles.

Said spoiler-shaped flap consists of a flap which follows the profile of the bottom edge of the goggles, and is set apart from said bottom edge at a distance of 3.5-5 mm at the top, of 4-6 mm at the bottom, and approximately 1 mm in the area of said outlet opening.

It has been seen, in fact, that the goggles made according to the present invention ensure:
- effective ventilation and effective de-misting without creating levels of turbulence inside the visor of the goggles;
- optimization of the ventilating effect within a speed range of 5-80 km/h;
- optimization of the ventilating effect also in the case of angles of incidence other than the neutral angle both laterally and longitudinally;
- optimization in the presence of a skiing helmet, the said helmet being able to restrict or regulate the flow in the side areas of the goggles.

According to the present invention, the geometry of the ventilation duct or passage is such as to prevent the creation of a pressure field that would render impossible a correct exchange of flow inside the visor. The arrangement according to the invention has as basic characteristics those of an air-inlet system set in an area of high static pressure and an air-outlet system set in an area of high speed of flow, typically the bottom part of the mask.

To verify the areas of positioning of the two inlet-outlet systems fluid-dynamic simulations were conducted that were based upon the geometry of the human face, and a wide range of geometries of goggles according to the known art were used.

As regards the outlet area, the areas of minimum dynamic pressure were found to be located at the bottom outlet edge of the mask, provided that internal channelling is made that enables the flow of air accelerated by the pressure gradient to cross an area of narrowing, further increasing the speed of the said air flow and locally creating conditions of low pressure.

As compared to the solutions according to the known art, the present invention enables areas of negative pressure to be obtained not in the side part, in which the flow has a direction and above all a magnitude of speed sufficient to create the effect of extraction, but where there is little possibility of control in terms of detachment of the flow in so far as the dynamic pressure of the air does not contribute to maintaining contact with the (variable) geometry of the face of the user.

The embodiment according to the invention guarantees a greater functionality that may range between 22 and the 34% with respect to the in-out solutions according to the known art, with a sensitivity to the variation of conditions that is clearly poorer.

The results are found to be optimized in a speed range of between 5 and 80 km/h, which is the range of the vast majority of skiers, downhillers and snowboarders.

The mask-type goggles according to the invention were subjected to an aerodynamic verification, namely computational fluid dynamics (CFD), to compare their functionality as against other mask-type goggles according to the known art. The findings are given below:
1. The mask-type goggles according to the invention ensure excellent ventilating functionality and an incoming air flow rate that is sufficient to guarantee a substantial improvement in de-misting rates. With conditioning at -20°C (a relative humidity 84%, and a temperature of 20°C), the time for complete de-misting was found to be 13.3 seconds as against the 24-67 seconds taken by masks according to the known art. The improvement obtained according to the invention is therefore equal to 94%-370% as compared to known masks.
2. The efficiency of air flow rate of the goggles according to the invention in the case of rotation of the user's head increases up to the angle of 38°, where there is the maximum flow rate in terms of amount of air processed; hence, well beyond the limit necessary for a snowboarder to ensure optimal ventilation.

Table 1 gives the values of the ventilation air flow rates as the angle of incidence varies for a mask produced according to the known art and currently available on the market, whilst Table 2 gives the values of the ventilation flow rates as the angle of incidence varies for the mask-type goggles built according to the present invention

**TABLE 1**

| Angle of incid. (deg.) | 0 | 5 | 10 | 15 | 20 | 25 | 30 | 35 | 40 |
|---|---|---|---|---|---|---|---|---|---|
| Speeds (km/h) | | | | | | | | | |
| 5 | 0.02 | 0.0204 | 0.0206 | 0.021 | 0.0214 | 0.0218 | 0.022 | 0.0207 | 0.02064 |
| 10 | 0.04 | 0.0408 | 0.0412 | 0.042 | 0.0428 | 0.0436 | 0.044 | 0.0414 | 0.04128 |
| 15 | 0.07 | 0.0714 | 0.0721 | 0.0735 | 0.0749 | 0.0763 | 0.077 | 0.07245 | 0.07224 |
| 20 | 0.09 | 0.0918 | 0.0927 | 0.0945 | 0.0963 | 0.0981 | 0.099 | 0.09315 | 0.09288 |
| 25 | 0.012 | 0.01224 | 0.01236 | 0.0126 | 0.01284 | 0.01308 | 0.0132 | 0.01242 | 0.01238 |
| 30 | 0.015 | 0.0153 | 0.01545 | 0.01575 | 0.01605 | 0.01635 | 0.0165 | 0.01552 | 0.01548 |
| 35 | 0.02 | 0.0204 | 0.0206 | 0.021 | 0.0214 | 0.0218 | 0.022 | 0.0207 | 0.02064 |
| 40 | 0.026 | 0.02652 | 0.02678 | 0.0273 | 0.02782 | 0.02834 | 0.0286 | 0.02691 | 0.02683 |
| 45 | 0.032 | 0.03264 | 0.03296 | 0.0336 | 0.03424 | 0.03488 | 0.0352 | 0.03312 | 0.03302 |
| 50 | 0.039 | 0.03978 | 0.04017 | 0.04095 | 0.04173 | 0.04251 | 0.0429 | 0.04036 | 0.04025 |
| 55 | 0.044 | 0.04488 | 0.04532 | 0.0462 | 0.04708 | 0.04796 | 0.0484 | 0.04554 | 0.04541 |
| 60 | 0.047 | 0.04794 | 0.04841 | 0.04935 | 0.05029 | 0.05123 | 0.0517 | 0.04864 | 0.04850 |
| 65 | 0.05 | 0.051 | 0.0515 | 0.0525 | 0.0535 | 0.0545 | 0.055 | 0.05175 | 0.0516 |
| 70 | 0.053 | 0.05406 | 0.05459 | 0.05565 | 0.05671 | 0.05777 | 0.0583 | 0.05486 | 0.05470 |

**TABLE 2**

| α Deg. | 0 | 5 | 10 | 15 | 20 | 25 | 30 | 35 | 40 |
|---|---|---|---|---|---|---|---|---|---|
| Speeds (km/h)) | | | | | | | | | |
| 5 | 0.023 | 0.02428 | 0.02493 | 0.0252 | 0.02525 | 0.02660 | 0.02794 | 0.02774 | 0.02828 |
| 10 | 0.046 | 0.04855 | 0.04985 | 0.0504 | 0.05050 | 0.05319 | 0.05588 | 0.05548 | 0.05655 |
| 15 | 0.0805 | 0.08497 | 0.08724 | 0.0882 | 0.08838 | 0.09309 | 0.09779 | 0.09708 | 0.09897 |
| 20 | 0.01035 | 0.10924 | 0.11217 | 0.1134 | 0.11363 | 0.11968 | 0.12573 | 0.12482 | 0.12725 |
| 25 | 0.0138 | 0.01457 | 0.01496 | 0.01512 | 0.01515 | 0.01596 | 0.01676 | 0.01664 | 0.01697 |
| 30 | 0.01725 | 0.01821 | 0.01869 | 0.0189 | 0.01894 | 0.01995 | 0.02095 | 0.02080 | 0.02121 |
| 35 | 0.023 | 0.02428 | 0.02493 | 0.0252 | 0.02525 | 0.02660 | 0.02794 | 0.02774 | 0.02828 |
| 40 | 0.0299 | 0.03156 | 0.03240 | 0.03276 | 0.03283 | 0.03457 | 0.03632 | 0.03606 | 0.03676 |
| 45 | 0.0368 | 0.03884 | 0.03988 | 0.04032 | 0.04040 | 0.04255 | 0.04470 | 0.04438 | 0.04524 |
| 50 | 0.04485 | 0.04734 | 0.04861 | 0.04914 | 0.04924 | 0.05186 | 0.05448 | 0.05409 | 0.05514 |
| 55 | 0.0506 | 0.05341 | 0.05484 | 0.05544 | 0.05555 | 0.05851 | 0.06147 | 0.06102 | 0.06221 |
| 60 | 0.05405 | 0.05705 | 0.05858 | 0.05922 | 0.05934 | 0.06250 | 0.06566 | 0.06518 | 0.06645 |
| 65 | 0.0575 | 0.06069 | 0.06231 | 0.063 | 0.06313 | 0.06649 | 0.06985 | 0.06934 | 0.07069 |
| 70 | 0.06095 | 0.06433 | 0.06605 | 0.06678 | 0.06692 | 0.07048 | 0.07404 | 0.07351 | 0.07493 |

As may be seen, the comparison of the effects obtained using the goggles according to the invention with those using goggles according to the known art indicates an increased functionality of approximately 17% on average, with peaks of 37% at high angles of incidence.

The mask-type goggles that form the subject of the present invention are described in what follows in a preferred but not exclusive practical embodiment with reference to the attached sheets of drawings provided purely by way of non-limiting illustration, and in which:

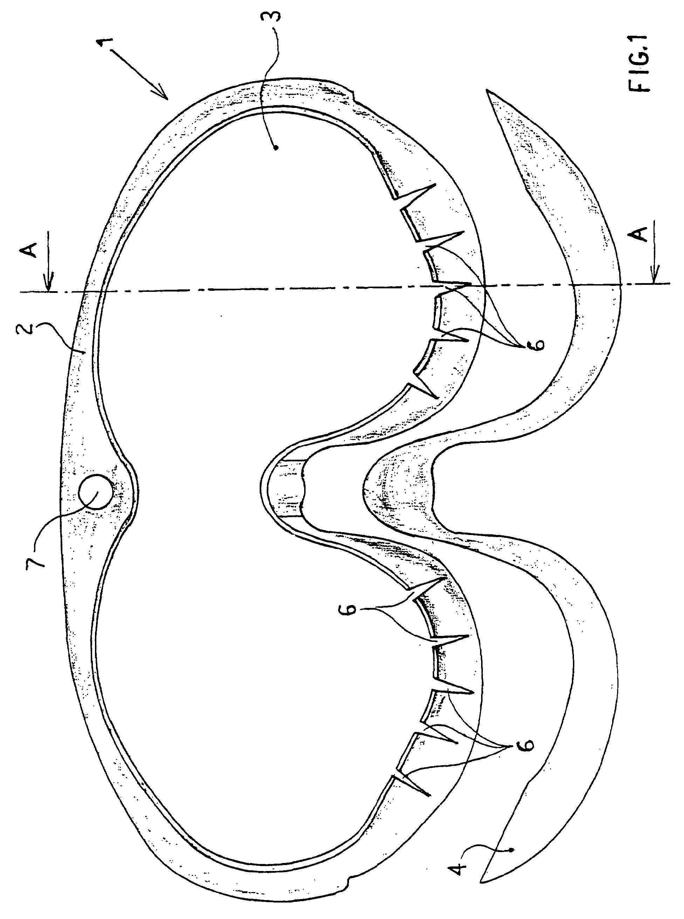

Figure 1 is a schematic front view of the mask-type goggle of the present invention, with the spoiler separate from the goggle itselves;

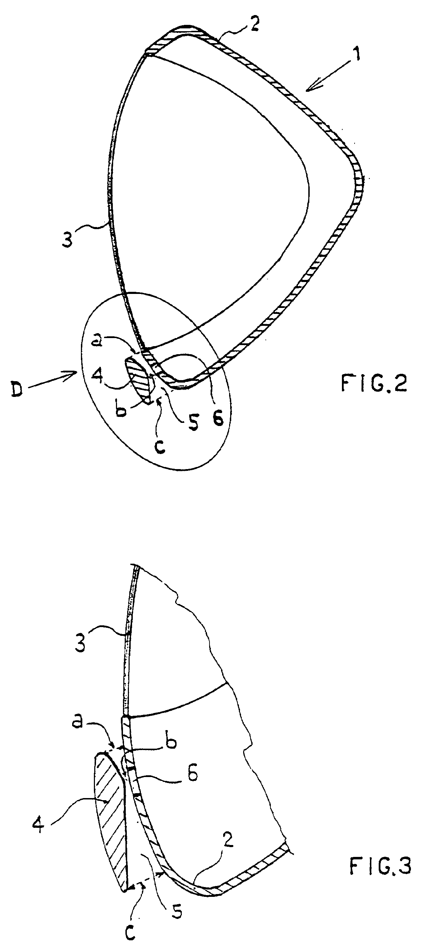

Figure 2 is a schematic sectional view of the goggle of Figure 1 taken along the line A-A, but with the spoiler in position instead of separate;
Figure 3 is a schematic view of an enlarged detail D of the goggle of Figure 2, with the spoiler in position.

With reference to the figures, the goggle 1 according to the present invention comprise the frame 2, the visor 3 and the spoiler 4. The spoiler 4 is shaped so that its internal profile, in combination with the external surface of the frame practically constitutes a venturi tube, designated by 5, such as to create a negative pressure at the outlet openings 6 present in the frame and thus create a ventilation of the space inside the goggles and an aspiration outwards of the air introduced into the internal space through the inlet opening 7 set in the upper edge of the frame 2, this air being saturated with humidity as a result of coming into contact with the user's face.

Preferably, to ensure an efficient suction effect, the spoiler 4 is positioned so that the distance a is 3.5-5 mm, the distance b is approximately 1 mm and the distance c is 4-6 mm.

Of course, the inlet and outlet openings may be of any number, shape and size, according to the shape and size of the goggles, also taking into account the aesthetic appearance of the goggles themselves. These openings are arranged preferably in the frame, but according to other embodiments may also be positioned at the front on the top and bottom edges of the visor, or else partly on the edges of the visor and partly in the frame, according to the shape and the aesthetic requirements of the goggle.

## Claims

1. Anti-misting (no-fogging) ventilated mask-type goggle for sporting activities, particularly for winter sports, such as skiing, cross-country skiing, snowboarding and the like, and for protective use in general, comprising a frame (2) and a visor (3) applied, also in such a way that it may be removed, to said frame (2), **characterized in that** it comprises at least one inlet opening (7) set at the front for the ventilation air and at least one outlet opening (6) for the ventilation air also set at the front and substantially on the same plane comprising said inlet opening, said outlet opening (6) being provided with a spoiler-shaped flap (4) which, in combination with the conformation of the external surface of the goggle in the proximity of said outlet opening (6), is designed to convey the flow of incident air so as to create a negative pressure at said outlet opening (6) and a suction from the space inside the goggles outwards.

2. The mask-type goggle according to Claim 1, **characterized in that** said inlet opening (7) is positioned in the upper edge of said frame (2) whilst said outlet opening (6) provided with spoiler (4) is positioned in the bottom edge of said frame.

3. The mask-type goggle according to Claim 1, **characterized in that** said inlet opening is positioned in the bottom edge of said frame (2), whilst said outlet opening provided with spoiler is positioned in the upper edge of said frame.

4. The mask-type goggle according to Claim 1, **characterized in that** said outlet opening (6) is made up of a plurality of holes or slits or notches arranged symmetrically with respect to the middle of the goggles.

5. The mask-type goggle according to Claim 1, **characterized in that** said spoiler-shaped flap (4) consists of a flap which follows the profile of the bottom edge of the goggles, and is set apart from said edge at a distance of 3.5-5 mm at the top, 4-6 mm at the bottom, and approximately 1 mm in the area of said outlet opening (6).
